# EUROPEAN PATENT SPECIFICATION

(11) **EP 4 361 125 B1**
(45) Date of publication and mention of the grant of the patent: **18.02.2026**
(21) Application number: 21956461.4
(22) Date of filing: 14.09.2021
(51) Int. Cl.: C07C 227/08, C07C 229/24, C07C 227/18

(54) **L-ASPARTIC ACID N, N-DIACETIC ACID TETRASODIUM SALT AND PREPARATION METHOD THEREFOR**
TETRANATRIUMSALZ VON L-ASPARAGINSÄURE N, N-DIESSIGSÄURE UND HERSTELLUNGSVERFAHREN DAFÜR
SEL DE TÉTRASODIUM D'ACIDE L-ASPARTIQUE N, N-DIACÉTIQUE ET SON PROCÉDÉ DE PRÉPARATION

(30) Priority: 08.09.2021 CN 202111048925
(43) Date of publication of application: 01.05.2024
(73) Proprietor: Hefei All-Plus Environmental Technology Co., Ltd., Hefei, Anhui 231602 (CN)
(72) Inventor: OU, Min, Hefei, Anhui 231602 (CN); MEI, Longyi, Hefei, Anhui 231602 (CN)
(74) Representative: Guardian IP Consulting I/S
(86) International application number: PCT/CN2021/118311
(87) International publication number: WO 2023/035284

(56) References cited:
- CN-A- 101 535 376
- CN-A- 109 400 492
- CN-A- 109 400 492
- CN-A- 112 430 194
- US-A1- 2021 054 146
- I. P. GORELOV ET AL: "SYNTHESIS AND COMPLEX-FORMIN G PROPERTIES OF COMPLEXONS DERIVED FROM DICARBOXYLIC ACIDS. V. SYNTHESIS OF COMPLEXONS DERIVED FROM SUCCINIC ACID", JOURNAL OF GENERAL CHEMISTRY OF THE USSR, vol. 49, no. 3, 1 January 1979 (1979-01-01), pages 573 - 576, XP055384591, ISSN: 0022-1279

## Description

### Cross Reference to Related Applications

The present disclosure claims priority to the Chinese patent application No. CN202111048925.X filed with the Chinese Patent Office on September 8, 2021, and entitled "L-aspartic Acid N,N-diacetic Acid Tetrasodium Salt and Preparation Method therefor".

### Technical Field

The present disclosure relates to the technical field of compounds, and in particular, to L-aspartic acid N,N-diacetic acid tetrasodium salt and a preparation method therefor.

### Background Art

Common chelating agents in prior art mainly include phosphates, hydroxycarboxylic acids, aminocarboxylic acids, and carboxylate-containing polymers. Among them, although phosphates have a better chelating effect, phosphorus contained causes great pollution to the environment. Sodium tripolyphosphate (STPP) is used more, but gradually fades out of the market due to the call for phosphorus restriction and phosphorus prohibition. The hydroxycarboxylic acids mainly include sodium gluconate, sodium citrate, etc., and they have a poor chelating performance for general metal ions, and thus a low cost performance. The acrylic polymers, which belong to high-molecular chelating agents, have the functions of thickening and flocculating in addition to the chelating ability, and generally settle in water after chelation, but are difficult to biodegrade. The aminocarboxylic acids mainly include ethylenediaminetetraacetic acid (EDTA), hydroxyethylethylenediaminetriacetic acid (HEDTA), diethylenetriaminepentaacetic acid (DTPA), nitrilotriacetic acid (NTA), iminodisuccinic acid (IDS), glutamic acid diacetic acid (GLDA), methylglycinediacetic acid (MGDA), etc. EDTA is not easy to biodegrade (OECD). NTA is potentially carcinogenic. IDS has a low chelating performance for metals. Raw materials of GLDA and MGDA are highly toxic and have high requirements to equipment during a fabrication process, such that they have the difficulty in industrialization, high production cost, and high product price, which often limits their wide use in actual life. The prior art also provides a biodegradable environmental-friendly chelating agent, L-aspartic acid N,N-diacetic acid tetrasodium salt (ASDA, Tetrasodium dicarboxymethy-I aspartate).

However, a preparation method for the L-aspartic acid N,N-diacetic acid tetrasodium salt provided in the prior art has a high cost, a low yield, and many by-products.

J. Gen. Chem. USSR Vol 49(3), pp 573-576 (1979) discloses the preparation of L-aspartic acid N,N-diacetic acid by Michael addition between maleic acid and iminodiacetic acid. CN-A-109 400 492 discloses the preparation of L-aspartic acid N,N-diacetic acid by the N-dialkylation of L-aspartic acid using ClCH₂CO₂H (without Michael addition being used).

### Summary

The present disclosure aims at providing an L-aspartic acid N,N-diacetic acid tetrasodium salt and a preparation method therefor, wherein the preparation method for L-aspartic acid N,N-diacetic acid tetrasodium salt has a low cost, a high yield, and few by-products.

The present disclosure is implemented as follows.

In a first aspect, the present disclosure provides a preparation method for L-aspartic acid N,N-diacetic acid tetrasodium salt, including:
hydrolyzing at least one of maleic anhydride, maleic acid, and fumaric acid to obtain a hydrolyzed salt solution; and
subjecting the hydrolyzed salt solution and glycine to a Michael addition reaction, and then carrying out a catalytic reaction with chloroacetic acid.

In an optional embodiment, the step of hydrolyzing at least one of the maleic anhydride, the maleic acid, and the fumaric acid specifically includes:
mixing at least one of the maleic anhydride, the maleic acid, and the fumaric acid with at least one of water, methanol, ethanol, and isopropanol, and then adding an alkali solution dropwise.

In an optional embodiment, a temperature is controlled at -40 °C~80 °C when adding the alkali solution dropwise.

In an optional embodiment, a temperature of hydrolyzing at least one of the maleic anhydride, the maleic acid, and the fumaric acid is 0 °C~75 °C.

In an optional embodiment, the step of subjecting the hydrolyzed salt solution and the glycine to the Michael addition reaction specifically includes:
to the hydrolyzed salt solution adding the glycine and an alkali solution, and reacting under conditions of a temperature of 45 °C-150 °C and a pressure of 0-10 MPa; after that, controlling temperature at 45 °C-100 °C, and then adding potassium iodide to react.

In an optional embodiment, the step of to the hydrolyzed salt solution adding the glycine and the alkali solution specifically includes:
mixing the glycine and the alkali solution, and then dropwise adding the mixed glycine and alkali solution to the hydrolyzed salt solution.

In an optional embodiment, the step of to the hydrolyzed salt solution adding the glycine and the alkali solution specifically includes:
adding the glycine and the alkali solution simultaneously to the hydrolyzed salt solution.

In an optional embodiment, the step of subjecting the hydrolyzed salt solution and the glycine to the Michael addition reaction, and then carrying out a catalytic reaction with the chloroacetic acid specifically includes:
dropwise adding the chloroacetic acid and alkali solution both in an equal molar weight to at least one raw material of the maleic anhydride, the maleic acid, and the fumaric acid simultaneously to a solution after the hydrolyzed salt solution and the glycine have undergone the Michael addition reaction

In an optional embodiment, the alkali solution includes at least one of a lithium hydroxide solution, a sodium hydroxide solution, and a potassium hydroxide solution.

In a second aspect, the present disclosure provides an L-aspartic acid N,N-diacetic acid tetrasodium salt, prepared by the preparation method for L-aspartic acid N,N-diacetic acid tetrasodium salt according to any one of the preceding embodiments.

The present disclosure has the following beneficial effects.

The preparation method for L-aspartic acid N,N-diacetic acid tetrasodium salt provided in the embodiments of the present disclosure includes: hydrolyzing at least one of maleic anhydride, maleic acid, and fumaric acid to obtain a hydrolyzed salt solution; and subjecting the hydrolyzed salt solution and glycine to a Michael addition reaction, and then carrying out a catalytic reaction with chloroacetic acid. Since the maleic anhydride, the maleic acid, the fumaric acid, the glycine, and the chloroacetic acid have wide sources and low costs, a preparation cost can be effectively reduced. As the reaction process includes the hydrolyzation, the Michael addition reaction, and the catalytic reaction, they are easy to operate and have high yields, and no toxic by-products are produced in the reaction process, thus the reaction process is highly safe and is environmentally friendly.

The preparation method for L-aspartic acid N,N-diacetic acid tetrasodium salt prepared in the embodiments of the present disclosure has a good chelating performance, high safety, and environmental friendliness.

### Brief Description of Drawings

In order to more clearly illustrate technical solutions of examples of the present disclosure, drawings which need to be used in the examples will be introduced below briefly. It should be understood that the drawings below merely show some examples of the present disclosure, and therefore should not be considered as limitation to the scope. Those ordinarily skilled in the art still could obtain other relevant drawings according to these drawings, without using any inventive efforts.
FIG. 1 is a graph of biodegradability detection results of ASDA prepared in Example 1 of the present disclosure tested in a stable sewage environment;
FIG. 2 is a comparison graph of solubility of the ASDA prepared in Example 1 of the present disclosure in water of different pH values;
FIG. 3 is a graph of solubility detection results of the ASDA prepared in Example 1 of the present disclosure in a concentrated sodium hydroxide solution;
FIG. 4 is a thermogravimetric analysis graph of the ASDA prepared in Example 1 of the present disclosure;
FIG. 5 is a comparison chart of chelating abilities of the ASDA prepared in Example 1 of the present disclosure and other chelating agents under the same condition; and
FIG. 6 is a comparison chart of efficiencies of removing calcium carbonate scales by the ASDA prepared in Example 1 of the present disclosure and other amino polymeric chelating agents.

### Detailed Description of Embodiments

In order to make the objectives, technical solutions, and advantages of the examples of the present disclosure clearer, the technical solutions in the examples of the present disclosure will be clearly and completely described below. If no specific conditions are specified in the examples, they are carried out under normal conditions or conditions recommended by manufacturers. If manufacturers of reagents or apparatuses used are not specified, they are conventional products commercially available.

A preparation method for L-aspartic acid N,N-diacetic acid tetrasodium salt (ASDA, Tetrasodium dicarboxymethy-I aspartate) provided in prior art, for example, using L-aspartate as a raw material for preparation, is likely to produce a large amount of by-products, which is prone to affect chelating performance of products, and limits industrial popularization thereof. Alternatively, there is also a method for producing ASDA by making amino acid have Strecker reaction with hydrocyanic acid and formaldehyde, while the raw materials are highly toxic and increase pressure on safety and environmental protection during storage and use, and meanwhile, the reaction process is accompanied by a large amount of by-products which are difficult to separate, such that application and industrialization of the products are restricted to a great extent.

The present disclosure provides a preparation method for L-aspartic acid N,N-diacetic acid tetrasodium salt, which has a low cost, a high yield, and few by-products.

The L-aspartic acid N,N-diacetic acid tetrasodium salt and the preparation method therefor are described in detail below.

The preparation method for L-aspartic acid N,N-diacetic acid tetrasodium salt provided in the present disclosure includes: hydrolyzing at least one of maleic anhydride, maleic acid, and fumaric acid to obtain a hydrolyzed salt solution; and subjecting the hydrolyzed salt solution and glycine to a Michael addition reaction, and then carrying out a catalytic reaction with chloroacetic acid. Since the maleic anhydride, the maleic acid, the fumaric acid, the glycine, and the chloroacetic acid have wide sources and low costs, a preparation cost can be effectively reduced. As the reaction process includes the hydrolyzation, the Michael addition reaction, and the catalytic reaction, they are easy to operate and have high yields, and no toxic by-products are produced in the reaction process, thus the reaction process is highly safe and is environmentally friendly.

It should be noted that the L-aspartic acid N,N-diacetic acid tetrasodium salt prepared by the method of the present disclosure has a good chelating performance, high safety, and environmental friendliness.

The step of hydrolyzing at least one of maleic anhydride, maleic acid, and fumaric acid specifically includes: mixing at least one of the maleic anhydride, the maleic acid, and the fumaric acid with at least one of water, methanol, ethanol, and isopropanol, and then adding an alkali solution dropwise.

Further, a temperature is controlled at -40-80 °C when adding the alkali solution dropwise; and after the dropwise addition, the mixture is stirred for a period of time for later use.

Further, a temperature of hydrolyzing at least one of the maleic anhydride, the maleic acid, and the fumaric acid is 0 °C~75 °C.

It should be noted that, when hydrolyzing at least one of the maleic anhydride, the maleic acid, and the fumaric acid, a weight ratio of a mixture of one or more of the maleic anhydride, the maleic acid, and the fumaric acid to one or more of the water, the methanol, the ethanol, and the isopropanol is approximately 40%-60%.

The step of subjecting the hydrolyzed salt solution and glycine to a Michael addition reaction specifically includes:
to the hydrolyzed salt solution adding the glycine and an alkali solution, and reacting under conditions of a temperature of 45 °C-150 °C and a pressure of 0-10 MPa, wherein reaction may last for 2-24 h; after that, controlling the temperature at 45 °C~100 °C, and then adding potassium iodide to react.

It should be noted that, a mass of the potassium iodide may account for 0.01-10% of the mixture of one or more of the maleic anhydride, the maleic acid, and the fumaric acid, or the mass of the potassium iodide further may account for 0.01-10% of the mixture of one or more of the maleic anhydride, the maleic acid, and the fumaric acid, and the mixture of one or more of the water, the methanol, the ethanol, and the isopropanol, etc., which is not specifically limited herein.

It should also be noted that, when the hydrolyzed salt solution reacts with the glycine and the alkali solution at a temperature greater than 100 °C, the temperature needs to be reduced to 45 °C-100 °C after the reaction, and then the potassium iodide is added for reaction.

The step of to the hydrolyzed salt solution adding glycine and an alkali solution specifically includes:
mixing the glycine and the alkali solution, and then dropwise adding the mixed glycine and alkali solution to the hydrolyzed salt solution; or adding the glycine and the alkali solution simultaneously to the hydrolyzed salt solution.

In the preparation method for L-aspartic acid N,N-diacetic acid tetrasodium salt in the present disclosure, when catalyzing the reaction with the chloroacetic acid, the method specifically includes: dropwise adding the chloroacetic acid and the alkali solution both in an equal molar weight to at least one raw material of the maleic anhydride, the maleic acid, and the fumaric acid simultaneously to a solution after the hydrolyzed salt solution and the glycine have undergone the Michael addition reaction.

Further, pH is controlled at 6-12 when the above reaction is in progress, and after the dropwise addition is ended, the reaction is carried out for 2-24 h in a thermal insulation manner.

Still further, a temperature of the reaction carried out in the thermal insulation manner is approximately 45 °C-100 °C.

The alkali solution in the present disclosure includes at least one of a lithium hydroxide solution, a sodium hydroxide solution, and a potassium hydroxide solution. Further, a mass concentration of the alkali solution may be 30%-50%, that is, the mass concentration of at least one of the lithium hydroxide solution, the sodium hydroxide solution, and the potassium hydroxide solution is 30%-50%.

A reaction formula of the preparation method for L-aspartic acid N,N-diacetic acid tetrasodium salt provided in the present disclosure includes:

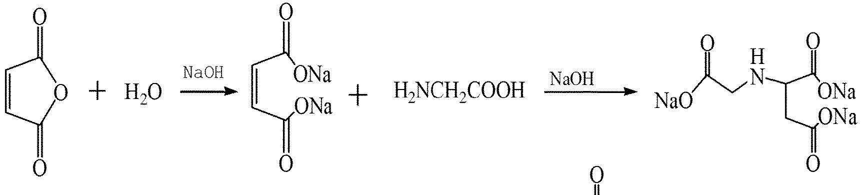

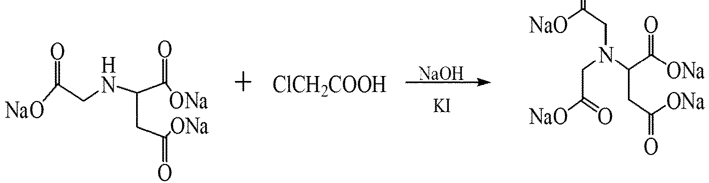

In the preparation method for L-aspartic acid N,N-diacetic acid tetrasodium salt provided in the present disclosure, at least one raw material of maleic anhydride, maleic acid, and fumaric acid is subjected to a hydrolysis neutralization reaction to generate maleate, the maleate undergoes the Michael addition reaction with glycine, and then continues to undergo catalytic and substitution reaction with chloroacetic acid to generate a biodegradable chelate, L-aspartic acid N,N-diacetic acid tetrasodium salt (ASDA). L-aspartic acid N,N-diacetic acid tetrasodium salt (ASDA) demonstrates particularly good overall chelating performance for calcium, magnesium, iron, copper, and other metals. In addition, the L-aspartic acid N,N-diacetic acid tetrasodium salt (ASDA) prepared by the preparation method of the present disclosure has very high solubility in both high-concentration aqueous alkali solution and aqueous acid solution, and also has excellent storage stability. The ASDA production method provided in the present disclosure is a process completely taking water as a system, has mild reaction conditions, and is substantially free of wastes during production. The preparation method for L-aspartic acid N,N-diacetic acid tetrasodium salt provided in the present disclosure is simple to operate and stable in process, has good product quality, good performance, huge application prospects and market economic benefits, and meanwhile is environmentally friendly.

The present disclosure is further described in detail below in combination with examples.

### Example 1

To a 5000 mL four-neck flask maleic anhydride 490 g was added, then deionized water 1200 g was added, and the mixture was hydrolyzed at 25 °C for 1 h. Then, pH value of reaction system was adjusted to about 6.0 with a NaOH solution having a mass fraction of 50%, with temperature being maintained to not exceed 45 °C. After dropwise addition of the NaOH solution was completed, to the reaction system glycine 375 g and 130 g of the NaOH solution having a mass fraction of 50% were added, the mixture was stirred for 45 min, heated to 85 °C, continued to be stirred for 3 h under a condition that pressure was 0.1 MPa. Potassium iodide 2 g was added, followed by stirring for 15 min. 940 g of chloroacetic acid solution having a mass fraction of 50% and 400 g of sodium hydroxide solution having a mass fraction of 50% were both simultaneously added dropwise to the above solution, pH being controlled at about 6. After the dropwise addition was completed, reaction was carried out in a thermal insulation manner at about 85 °C for 5 h. Resultant was cooled to obtain a solution of L-aspartic acid N,N-diacetic acid tetrasodium salt, with a content of 85.6% and a yield of 98.3%.

### Example 2

To a 5000 mL four-neck flask maleic acid 530 g was added, then deionized water 1200 g was added, and the mixture was hydrolyzed at 25 °C for 1 h. Then, pH value of reaction system was adjusted to about 6.5 with a NaOH solution having a mass fraction of 50%, with temperature being maintained to not exceed 45 °C. After dropwise addition of the NaOH solution was completed, to the reaction system glycine 385 g and 130 g of the NaOH solution having a mass fraction of 50% were added, the mixture was stirred for 45 min, heated to 75 °C, continued to be stirred for 3 h under a condition that pressure was 0.5 MPa. Potassium iodide 2 g was added, followed by stirring for 15 min. 940 g of chloroacetic acid solution having a mass fraction of 50% and 400 g of sodium hydroxide solution having a mass fraction of 50% were both simultaneously added dropwise to the above solution, pH being controlled at about 12. After the dropwise addition was completed, reaction was carried out in a thermal insulation manner at about 75 °C for 5 h. Resultant was cooled to obtain a solution of L-aspartic acid N,N-diacetic acid tetrasodium salt, with a content of 86.2% and a yield of 99%.

### Example 3

To a 5000 mL four-neck flask fumaric acid 490 g was added, then deionized water 1200 g was added, and the mixture was hydrolyzed at 25 °C for 1 h. Then, pH value of reaction system was adjusted to about 6.3 with a NaOH solution having a mass fraction of 50%, with temperature being maintained to not exceed 45 °C. After dropwise addition of the NaOH solution was completed, to the reaction system glycine 375 g and 130 g of the NaOH solution having a mass fraction of 50% were added, the mixture was stirred for 45 min, heated to 65 °C, continued to be stirred for 4 h under a condition that pressure was 3 MPa. Potassium iodide 5 g was added, followed by stirring for 15 min. 1040 g of chloroacetic acid solution having a mass fraction of 50% and 420 g of sodium hydroxide solution having a mass fraction of 50% were both simultaneously added dropwise to the above solution, pH being controlled at about 8. After the dropwise addition was completed, reaction was carried out in a thermal insulation manner at about 65 °C for 15 h. Resultant was cooled to obtain a solution of L-aspartic acid N,N-diacetic acid tetrasodium salt, with a content of 85.6% and a yield of 97.6%.

### Example 4

To a 5000 mL four-neck flask maleic anhydride 490 g was added, then deionized water 1200 g was added, and the mixture was hydrolyzed at 25 °C for 1 h. Then, pH value of reaction system was adjusted to about 6.2 with a KOH solution having a mass fraction of 30%, with temperature being maintained to not exceed 45 °C. After dropwise addition of the KOH solution was completed, to the reaction system glycine 375 g and 148 g of the KOH solution having a mass fraction of 30% were added, the mixture was stirred for 45 min, heated to 105 °C, continued to be stirred for 3 h under a condition that pressure was 0.3 MPa. Potassium iodide 2 g was added, followed by stirring for 15 min. 940 g of chloroacetic acid solution having a mass fraction of 50% and 610 g of sodium hydroxide solution having a mass fraction of 30% were both simultaneously added dropwise to the above solution, pH being controlled at about 10. After the dropwise addition was completed, reaction was carried out in a thermal insulation manner at about 95 °C for 5 h. Resultant was cooled to obtain a solution of L-aspartic acid N,N-diacetic acid tetrasodium salt, with a content of 80.6% and a yield of 95.2%.

### Example 5

To a 5000 mL four-neck flask maleic anhydride 490 g was added, then deionized water 1200 g and methanol 400 g were added, and the mixture was hydrolyzed at 25 °C for 1 h. Then, pH value of reaction system was adjusted to about 6.3 with a NaOH solution having a mass fraction of 50%, with temperature being maintained to not exceed 45 °C. After dropwise addition of the NaOH solution was completed, to the reaction system glycine 375 g and 130 g of the NaOH solution having a mass fraction of 50% were added, the mixture was stirred for 45 min, heated to 90 °C, continued to be stirred for 6 h under a condition that pressure was 0.5 MPa. Potassium iodide 2 g was added, followed by stirring for 15 min. 940 g of chloroacetic acid solution having a mass fraction of 50% and 400 g of sodium hydroxide solution having a mass fraction of 50% were both simultaneously added dropwise to the above solution, pH being controlled at about 7. After the dropwise addition was completed, reaction was carried out in a thermal insulation manner at about 80 °C for 10 h. Resultant was cooled to obtain a solution of L-aspartic acid N,N-diacetic acid tetrasodium salt, with a content of 84.5% and a yield of 97.8%.

### Example 6

To a 5000 mL four-neck flask 490 g of a mixture of maleic anhydride, maleic acid, and fumaric acid was added, then 1200 g of a mixture of deionized water, ethanol, methanol, and isopropanol was added, and the mixture was hydrolyzed at 75 °C for 1 h. Then, pH value of reaction system was adjusted to about 6.0 with a LiOH solution having a mass fraction of 40%, with temperature being maintained to not exceed 80 °C. After dropwise addition of the LiOH solution was completed, to the reaction system glycine 375 g and 130 g of the LiOH solution having a mass fraction of 40% were added, the mixture was stirred for 45 min, heated to 150 °C, continued to be stirred for 3 h under a condition that pressure was 5 MPa. The temperature was reduced to 45 °C, and potassium iodide 2 g was added, followed by stirring for 15 min. 940 g of chloroacetic acid solution having a mass fraction of 50% and 400 g of the LiOH solution having a mass fraction of 40% were both simultaneously added dropwise to the above solution, pH being controlled at about 9. After the dropwise addition was completed, reaction was carried out in a thermal insulation manner at about 45 °C for 2 h. Resultant was cooled to obtain a solution of L-aspartic acid N,N-diacetic acid tetrasodium salt.

### Example 7

To a 5000 mL four-neck flask 490 g of a mixture of maleic acid and fumaric acid was added, then 1200 g of a mixture of methanol and isopropanol was added, and the mixture was hydrolyzed at 5 °C for 1 h. Then, pH value of reaction system was adjusted to about 6.4 with a KOH solution having a mass fraction of 38%, with temperature being maintained at about 20 °C. After dropwise addition of the KOH solution was completed, to the reaction system glycine 375 g and 130 g of the KOH solution having a mass fraction of 38% were added, the mixture was stirred for 45 min, heated to 45 °C, continued to be stirred for 3 h under a condition that pressure was 10 MPa. Potassium iodide 2 g was added, followed by stirring for 15 min. 940 g of chloroacetic acid solution having a mass fraction of 50% and 400 g of the KOH solution having a mass fraction of 38% were both simultaneously added dropwise to the above solution, pH being controlled at about 7.5. After the dropwise addition was completed, reaction was carried out in a thermal insulation manner at about 45 °C for 24 h. Resultant was cooled to obtain a solution of L-aspartic acid N,N-diacetic acid tetrasodium salt.

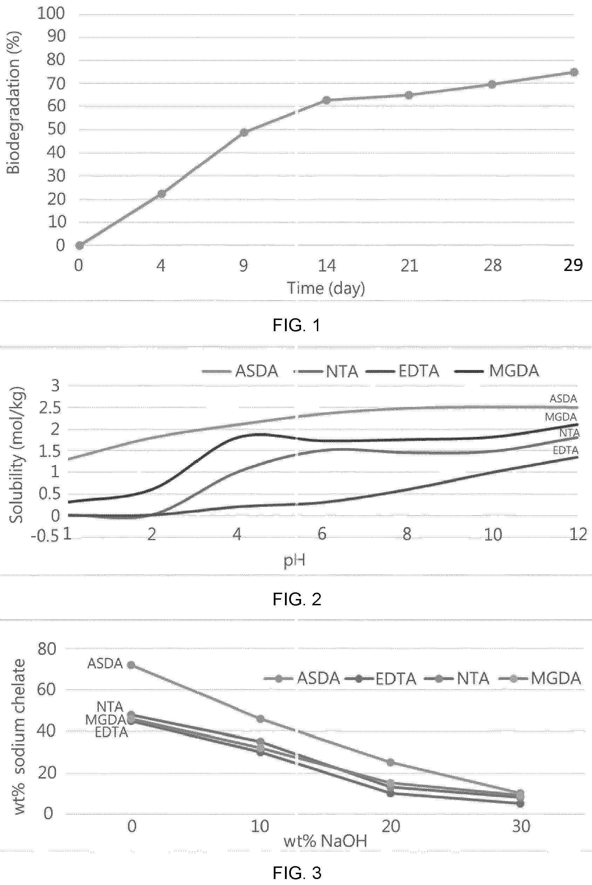

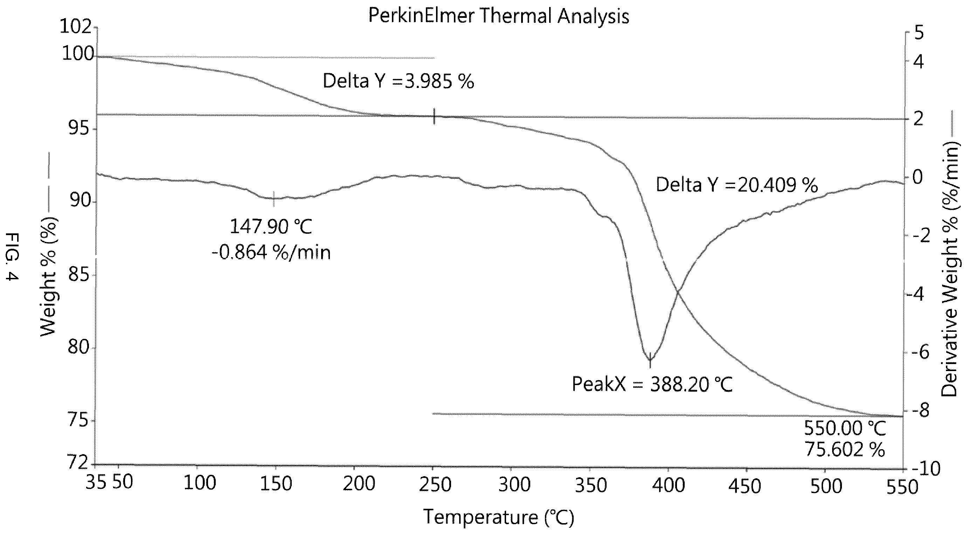

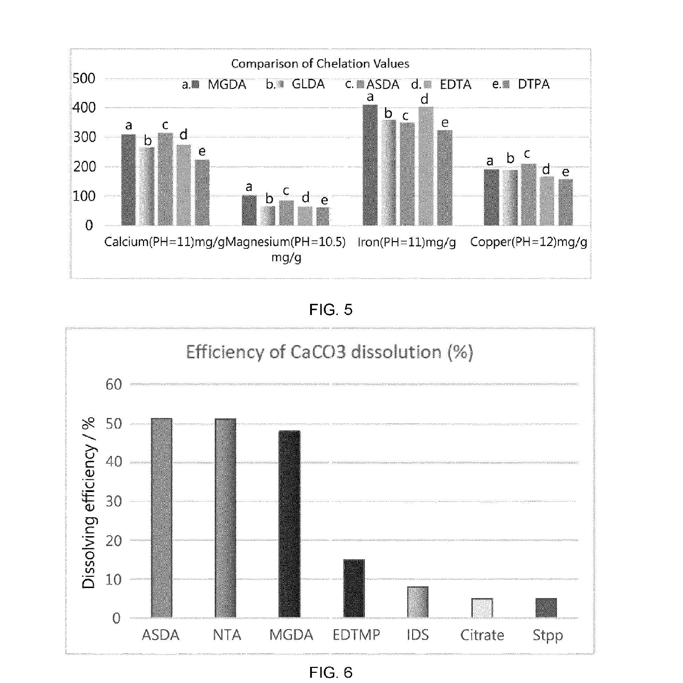

The ASDA is a colorless transparent liquid at room temperature. Anions of the ASDA may form coordinative geometrical structures with metal cations. The ASDA has a very strong chelating ability for calcium, magnesium, iron, copper, and other transition metal ions, and the cost performance has exceeded those of EDTA and other biodegradable chelating agents of the same type to a great extent. In addition, the ASDA has a relatively high dispersion capability, and may have a better effect when being used in combination with other chelating dispersants. Meanwhile, the ASDA can be used as a heavy-metal-ion cleaning agent, for example, it can be applied to a civilian detergent, an industrial cleaning agent, a dyeing adjuvant, a dyeing and finishing process, textile industry, paper industry, a photosensitive material, ceramic industry, electroplating industry, and extraction of heavy-metal contaminants from soil, as well as a dirt dispersion agent in the field of conventional industrial circulating water.
1. The biodegradability of the L-aspartic acid N,N-diacetic acid tetrasodium salt (ASDA) prepared by the preparation method provided in Example 1 was detected. The detection was carried out by a third-party detection mechanism. According to OECD 301B standard, 28-day biodegradability of the ASDA in sewage reaches 60% or more, as shown in FIG. 1 in detail.
2. Results of high solubility of the L-aspartic acid N,N-diacetic acid tetrasodium salt (ASDA) prepared by the preparation method provided in Example 1 in a wide pH range are shown in FIG. 2 and FIG. 3. In the above, ASDA has high solubility in low pH, indicating that the ASDA can be used for preparing an acidic cleaning agent; and the ASDA has high solubility under a strong alkali condition, which is more in line with requirements of high-pH and high-concentration washing environments. It should be noted that the ASDA, with high solubility, can be formulated into products having a high solid content, thereby facilitating the reduction of packaging when preparing ASDA-containing products, and further addressing the problem of packaging waste. It can also correspondingly reduce transportation and warehousing costs, and alleviate the problem of high transportation and warehousing costs.
3. Thermal stability and application environment adaptability of the L-aspartic acid N,N-diacetic acid tetrasodium salt (ASDA) prepared by the preparation method provided in Example 1 were detected. An ASDA thermogravimetric analysis graph is shown in FIG. 4. As can be seen from the detection, EDTA and NTA are thermally more stable (>150 °C) than ASDA. The thermal stability of ASDA-Na4 powder was measured by thermogravimetric analysis (TGA) and differential scanning calorimetry (DSC). ASDA solid lost all the moisture (about 4%) at about 250 °C, and began to rapidly decompose above 380 °C.
4. The chelating ability of the L-aspartic acid N,N-diacetic acid tetrasodium salt (ASDA) prepared by the preparation method provided in Example 1 was detected. Results show that comprehensive performance of the ASDA in chelating metal ions under the same condition is excellent (see FIG. 5).
5. The ASDA can also be used for preventing water scale and precipitation and removing water scale. By comparing the efficiencies of removing calcium carbonate water scales by the L-aspartic acid N,N-diacetic acid tetrasodium salt (ASDA) prepared by the preparation method provided in Example 1 and by other amino polymeric chelating agents, it shows that the ASDA is the best biodegradable chelate for removing CaCO₃ scales (see FIG. 6).

In conclusion, the preparation method for L-aspartic acid N,N-diacetic acid tetrasodium salt in the present disclosure can prepare the L-aspartic acid N,N-diacetic acid tetrasodium salt with good chelating performance, high safety. and environmental friendliness. Moreover, the raw materials of the preparation method in the present disclosure have wide sources and low costs, thus the preparation cost can be effectively reduced. As the reaction process includes hydrolyzation, Michael addition reaction, and catalytic reaction, they are easy to operate and have high yields, and no toxic by-products are produced in the reaction process, thus the reaction process is highly safe and is environmentally friendly.

## Claims

1. A preparation method for L-aspartic acid N,N-diacetic acid tetrasodium salt, **characterized by** comprising:
hydrolyzing at least one of maleic anhydride, maleic acid, and fumaric acid to obtain a hydrolyzed salt solution; and
subjecting the hydrolyzed salt solution and glycine to a Michael addition reaction, and then carrying out a catalytic reaction with chloroacetic acid

2. The preparation method for L-aspartic acid N,N-diacetic acid tetrasodium salt according to claim 1, wherein the step of hydrolyzing at least one of maleic anhydride, maleic acid, and fumaric acid comprises:
mixing at least one of the maleic anhydride, the maleic acid, and the fumaric acid with at least one of water, methanol, ethanol, and isopropanol, and then adding an alkali solution dropwise.

3. The preparation method for L-aspartic acid N,N-diacetic acid tetrasodium salt according to claim 2, wherein a temperature is controlled at -40 °C~80 °C when adding the alkali solution dropwise.

4. The preparation method for L-aspartic acid N,N-diacetic acid tetrasodium salt according to any one of claims 1-3, wherein a temperature of hydrolyzing at least one of the maleic anhydride, the maleic acid, and the fumaric acid is 0 °C~75 °C.

5. The preparation method for L-aspartic acid N,N-diacetic acid tetrasodium salt according to claim 1, wherein the step of subjecting the hydrolyzed salt solution and glycine to a Michael addition reaction comprises:
to the hydrolyzed salt solution adding the glycine and an alkali solution, and reacting under conditions of a temperature of 45 °C-150 °C and a pressure of 0-10 MPa; then controlling a temperature at 45 °C-100 °C, and then adding potassium iodide to react.

6. The preparation method for L-aspartic acid N,N-diacetic acid tetrasodium salt according to claim 5, wherein the step of to the hydrolyzed salt solution adding the glycine and an alkali solution comprises:
mixing the glycine and the alkali solution, and then dropwise adding mixed glycine and alkali solution to the hydrolyzed salt solution.

7. The preparation method for L-aspartic acid N,N-diacetic acid tetrasodium salt according to claim 5, wherein the step of to the hydrolyzed salt solution adding the glycine and an alkali solution comprises:
adding the glycine and the alkali solution simultaneously to the hydrolyzed salt solution.

8. The preparation method for L-aspartic acid N,N-diacetic acid tetrasodium salt according to claim 1, wherein the step of subjecting the hydrolyzed salt solution and glycine to a Michael addition reaction, and then carrying out a catalytic reaction with chloroacetic acid comprises:
dropwise adding the chloroacetic acid and an alkali solution, both in an equal molar weight to at least one raw material of the maleic anhydride, the maleic acid, and the fumaric acid, simultaneously to a solution after the hydrolyzed salt solution and the glycine have undergone the Michael addition reaction

9. The preparation method for L-aspartic acid N,N-diacetic acid tetrasodium salt according to claim 2, 5, or 8, wherein the alkali solution comprises at least one of a lithium hydroxide solution, a sodium hydroxide solution, and a potassium hydroxide solution.

## Patentansprüche

1. Herstellungsverfahren für L-Asparaginsäure-N,N-diessigsäure-Tetranatriumsalz, **dadurch gekennzeichnet, dass** es Folgendes umfasst:
Hydrolysieren mindestens einer von Maleinsäureanhydrid, Maleinsäure oder Fumarsäure, um eine hydrolysierte Salzlösung zu erhalten; und
Unterziehen der hydrolysierten Salzlösung und Glycin einer Michael-Additionsreaktion und anschließend Ausführen einer katalytischen Reaktion mit Chloressigsäure.

2. Herstellungsverfahren für L-Asparaginsäure-N,N-diessigsäure-Tetranatriumsalz nach Anspruch 1, wobei der Schritt zum Hydrolysierenmindestens einer von Maleinsäureanhydrid, Maleinsäure und Fumarsäure Folgendes umfasst:
Mischen mindestens eines des Maleinsäureanhydrids, der Maleinsäure und der Fumarsäure mit mindestens einem von Wasser, Methanol, Ethanol oder Isopropanol und anschließend tropfenweises Zugeben einer Alkalilösung.

3. Herstellungsverfahren für L-Asparaginsäure-N,N-diessigsäure-Tetranatriumsalz nach Anspruch 2, wobei die Temperatur beim tropfenweisen Zugeben der Alkalilösung auf -40 °C~80 °C geregelt wird.

4. Herstellungsverfahren für L-Asparaginsäure-N,N-diessigsäure-Tetranatriumsalz nach einem der Ansprüche 1-3, wobei eine Hydrolysetemperatur mindestens eines des Maleinsäureanhydrids, der Maleinsäure und der Fumarsäure 0 °C~75 °C beträgt.

5. Herstellungsverfahren für L-Asparaginsäure-N,N-diessigsäure-Tetranatriumsalz nach Anspruch 1, wobei der Schritt zum Unterziehen der hydrolysierten Salzlösung und Glycin einer Michael-Additionsreaktion Folgendes umfasst:
Zugeben zu der hydrolysierten Salzlösung des Glycins und einer Alkalilösung und Umsetzen unter Bedingungen einer Temperatur von 45 °C-150 °C und eines Druck von 0-10 MPa; anschließend Regeln der Temperatur auf 45 °C-100 °C und anschließend Zugeben von Kaliumiodid zur Reaktion.

6. Herstellungsverfahren für L-Asparaginsäure-N,N-diessigsäure-Tetranatriumsalz nach Anspruch 5, wobei der Schritt des Zugebens zu der hydrolysierten Salzlösung des Glycins und einer Alkalilösung Folgendes umfasst:
Mischen des Glycins und der Alkalilösung und anschließend tropfenweises Zugeben der gemischten Glycin-Alkali-Lösung zur hydrolysierten Salzlösung.

7. Herstellungsverfahren für L-Asparaginsäure-N,N-diessigsäure-Tetranatriumsalz nach Anspruch 5, wobei der Schritt des Zugebens zu der hydrolysierten Salzlösung des Glycins und einer Alkalilösung Folgendes umfasst:
gleichzeitiges Zugeben des Glycins und der Alkalilösung zu der hydrolysierten Salzlösung.

8. Herstellungsverfahren für L-Asparaginsäure-N,N-diessigsäure-Tetranatriumsalz nach Anspruch 1, wobei der Schritt zum Unterziehen der hydrolysierten Salzlösung und Glycin einer Michael-Additionsreaktion und anschließendem Ausführen einer katalytischen Reaktion mit Chloressigsäure Folgendes umfasst:
tropfenweises Zugeben der Chloressigsäure und einer Alkalilösung, jeweils in gleichem molaren Verhältnis zu mindestens einem Rohstoff des Maleinsäureanhydrids, der Maleinsäure und der Fumarsäure, gleichzeitig zu einer Lösung, nachdem die hydrolysierte Salzlösung und das Glycin die Michael-Additionsreaktion durchlaufen haben.

9. Herstellungsverfahren für L-Asparaginsäure-N,N-Diessigsäure-Tetranatriumsalz nach Anspruch 2, 5 oder 8, wobei die Alkalilösung mindestens eine einer Lithiumhydroxidlösung, einer Natriumhydroxidlösung und einer Kaliumhydroxidlösung umfasst.

## Revendications

1. Procédé de préparation du sel tétrasodique de l'acide L-aspartique-N,N-diacétique, **caractérisé en ce qu'**il comprend :
l'hydrolyse d'au moins l'un des éléments parmi l'anhydride maléique, l'acide maléique et l'acide fumarique pour obtenir une solution saline hydrolysée ; et
le fait de soumettre la solution saline hydrolysée et la glycine à une réaction d'addition de Michael, puis la réalisation d'une réaction catalytique avec l'acide chloroacétique.

2. Procédé de préparation du sel tétrasodique de l'acide L-aspartique-N,N-diacétique selon la revendication 1, dans lequel l'étape consistant à hydrolyser au moins l'un des éléments parmi l'anhydride maléique, l'acide maléique et l'acide fumarique comprend :
le mélange d'au moins l'un des éléments parmi l'anhydride maléique, l'acide maléique et l'acide fumarique avec au moins l'un des éléments parmi l'eau, le méthanol, l'éthanol et l'isopropanol, puis l'ajout goutte à goutte d'une solution alcaline.

3. Procédé de préparation du sel tétrasodique de l'acide L-aspartique-N,N-diacétique selon la revendication 2, dans lequel la température est maintenue à -40 °C~80 °C lors de l'ajout goutte à goutte de la solution alcaline.

4. Procédé de préparation du sel tétrasodique de l'acide L-aspartique-N,N-diacétique selon l'une quelconque des revendications 1-3, dans lequel la température d'hydrolyse d'au moins l'un des éléments parmi l'anhydride maléique, l'acide maléique et l'acide fumarique est de 0 °C~75 °C.

5. Procédé de préparation du sel tétrasodique de l'acide L-aspartique-N,N-diacétique selon la revendication 1, dans lequel l'étape consistant à soumettre la solution saline hydrolysée et la glycine à une réaction d'addition de Michael comprend :
l'ajout, à la solution saline hydrolysée, de la glycine et d'une solution alcaline, et la mise en réaction dans des conditions d'une température de 45 °C~150 °C et d'une pression de 0 à 10 MPa ; puis, le maintien de la température à 45 °C-100 °C, puis, l'ajout d'iodure de potassium pour démarrer la réaction.

6. Procédé de préparation du sel tétrasodique de l'acide L-aspartique-N,N-diacétique selon la revendication 5, dans lequel l'étape consistant à ajouter, à la solution saline hydrolysée, la glycine et une solution alcaline comprend :
le mélange de la glycine et de la solution alcaline, puis l'ajout goutte à goutte du mélange de glycine et de solution alcaline à la solution saline hydrolysée.

7. Procédé de préparation du sel tétrasodique de l'acide L-aspartique-N,N-diacétique selon la revendication 5, dans lequel l'étape consistant à ajouter, à la solution saline hydrolysée, la glycine et une solution alcaline comprend :
l'ajout simultané de la glycine et de la solution alcaline à la solution saline hydrolysée.

8. Procédé de préparation du sel tétrasodique de l'acide L-aspartique-N,N-diacétique selon la revendication 1, dans lequel l'étape consistant à soumettre la solution saline hydrolysée et la glycine à une réaction d'addition de Michael, puis à réaliser une réaction catalytique avec l'acide chloroacétique comprend :
l'ajout goutte à goutte de l'acide chloroacétique et d'une solution alcaline, toutes deux selon un poids équimolaire à au moins une matière première parmi l'anhydride maléique, l'acide maléique et l'acide fumarique, simultanément à une solution après que la solution saline hydrolysée et la glycine ont subi la réaction d'addition de Michael.

9. Procédé de préparation du sel tétrasodique de l'acide L-aspartique-N,N-diacétique selon la revendication 2, 5 ou 8, dans lequel la solution alcaline comprend au moins une solution parmi une solution d'hydroxyde de lithium, une solution d'hydroxyde de sodium et une solution d'hydroxyde de potassium.
